# EUROPÄISCHE PATENTSCHRIFT

(11) **EP 2 257 246 B1**
(45) Veröffentlichungstag und Bekanntmachung des Hinweises auf die Patenterteilung: **19.01.2022**
(21) Anmeldenummer: 08857733.3
(22) Anmeldetag: 06.11.2008
(51) Int. Cl.: A61F 2/44

(54) **BANDSCHEIBENPROTHESE**
INTERVERTEBRAL DISK PROSTHESIS
PROTHÈSE DE DISQUE INTERVERTÉBRAL

(30) Priorität: 04.12.2007 DE 102007058304
(43) Veröffentlichungstag der Anmeldung: 08.12.2010
(73) Patentinhaber: Global Medical Consulting Gmbh, 94327 Bogen (DE)
(72) Erfinder: BERTAGNOLI, Rudolf, 94315 Straubing (DE)
(86) Internationale Anmeldenummer: PCT/DE2008/001829
(87) Internationale Veröffentlichungsnummer: WO 2009/071044

(56) Entgegenhaltungen:
- WO-A-2004/054479
- WO-A-2004/071282
- WO-A-2005/041818
- FR-A- 2 877 833
- US-A1- 2005 143 824
- US-A1- 2006 293 752
- US-A1- 2007 010 826
- US-A1- 2007 225 810

## Beschreibung

Die Erfindung bezieht sich auf eine Bandscheibenprothese bestehend aus gelenkigen Prothesenkomponenten und Mitteln, die die Beweglichkeit begrenzen, wobei die Begrenzungsmittel derart ausgebildet sind, dass sie die Beuge-Extensionsbewegung und/ oder die Seitenneigung (13) und/oder die Rotation um die Hochachse asymmetrisch begrenzen.

Es sind Bandscheibenprothesen bekannt, bei denen Prothesenkomponenten Bewegungen in mehreren Richtungen erlauben, so daß ein Patient, der eine oder mehrere Prothesen dieser Art als Bandscheibenersatz trägt, seine ursprüngliche Bewegungsfreiheit weitgehend beibehalten kann.

Gemäß US2004/0158328A1, WO 2004/019828 A1, EP 1 124 509 B1 und DE 42 08 116 C2 werden Begrenzungen der Rotation um die Hochachse durch formgebende Maßnahmen vorgeschlagen, indem die Gleitflächen für die Seitenneigungen einerseits und für die Beuge/Extensionsneigung andererseits unterschiedliche Krümmungsradien erhalten. Dieses bedingt jedoch komplizierte Formgestaltungen der Prothesenkomponenten.

Um die Bewegungsfreiheitsgrade einer natürlichen Bandscheide zu imitieren wird gemäß DE 103 61 772 A1 eine Prothesenzwischenkomponente vorgeschlagen, die zwischen Deck- und Grundplatte Translations- und Rotationsbewegungen relativ zu den Platten ausführen kann. Damit sind jedoch Bewegungen nicht optimal begrenzbar.

Aus US 2005/010826 A1 sind Mittel zur Begrenzung der Beweglichkeit bekannt, die aus Begrenzungsarme bestehen, deren Elastizität verändert oder entsprechend abgestimmt werden kann, derart dass die Elastizität der Arme in der Seitenneigungsebene höher oder geringer ist, als die der Arme in der Beuge-Extensions-Bewegungsebene. D. h. die Elastizität beider Arme einer Ebene ist gleich.

Mittel zur Begrenzung der Beweglichkeit in verschiedenen Ebenen sind aus der US 2005/0143824 A1 bekannt. Die einzelnen Begrenzungspuffer bestehen aus jeweils Materialien mit unterschiedlichen elastischen Eigenschaften. Hiermit soll eine elastische Feinabstimmung der Begrenzungsmittel zur besseren Anpassung an die Bedürfnisse eines Patienten erreicht werden, wobei auch eine asymmetrische Begrenzungen in einer Bewegungsebene angedacht ist.

Die Beweglichkeit des Implantats wird für den individuellen Patienten angepasst in jeder Bewegungsrichtung gezielt begrenzt, d.h. die Beuge-Extensionsbewegung und/oder die Seitenneigung und/oder die Rotation um die Hochachse werden symmetrisch, wo möglich bzw. nötig auch asymmetrisch begrenzt. Es können damit auch Bewegungsfehlstellungen besser korrigiert werden.

Die Verwendung von elastischen Dämpfern ist auch aus der DE 203 15 611 U1 bekannt, jedoch handelt es sich hierbei um symmetrische Elemente, die gleiche Rückstellkräfte auf die ausgelenkten Prothesenkomponente unabhängig von der Bewegungsrichtung ausüben. Elastische Dämpfer können jedoch durch die starke Belastung im menschlichen Körper ihre Eigenschaften über die Zeit verändern oder gar verlieren.

Der Erfindung liegt die Aufgabe zugrunde, Prothesen mit Bewegungsbegrenzungsmittel zu verbessern. Die Aufgabe wird durch die Merkmale des Anspruchs 1 gelöst.

Zur Durchführung der erfindungsgemäßen Prothese eignen sich die fertigungstechnisch einfachen Bandscheibenprothesen mit Kugel-Pfannensystem, die dreidimensional beweglich, d.h. dreh- und schwenkbar sind. Gezielt werden Begrenzungsmittel zur gleichen oder ungleichen Begrenzung der Beuge- und Extensionsbewegung einerseits und Seitenneigung in beide Richtungen andererseits verwendet. Die Fixierung der Dämpfer erfolgt an den Wirbelkörpern oder an künstlichen Wirbelendplatten bzw. Appositionsplatten.

Gemäß einer bevorzugten Ausgestaltung werden die künstlichen Wirbelendplatten getrennt von der Prothese mit den Wirbelkörpern derart verbunden, dass sie auf Dauer verankert bleiben und als Halterung für die Prothese und Bewegungsbegrenzungsmittel dienen. Dieses hat den Vorteil, dass ein notwendiger Austausch der Prothese und/oder eines oder mehrerer Bewegungsbegrenzungsmittel nur eine mechanische Lösung und wieder Anbindung an die künstlichen Wirbelendplatten und nicht am Knochen bedeutet. Damit wird der Operationsvorgang erleichtert und beschleunigt sowie die Knochensubstanz geschont.

Eine einseitige Verbindung der Dämpfer, z.B. an nur einer künstlichen Wirbelendplatte oder einem Wirbelkörper bewirkt, dass diese Dämpfer keinen Einfluß auf die Rotationsbewegung um die Hochachse haben, so dass deren Begrenzung unabhängig von der Beugebewegung ebenfalls gezielt gewählt werden kann. Hierfür könnten beispielsweise Zuggurtungsbänder unterschiedlicher Stärken verwendet werden, die über Kreuz an beiden künstlichen Wirbelendplatten oder Wirbelkörpern fixiert die Rotation um die Hochachse nach rechts und links asymmetrisch Begrenzt oder mit unterschiedlichen Rückstellkräften belegen.

Eine einfache Ausführung mit elastischen Elementen besteht erfindungsgemäß in einem U-förmigen Dämpfer mit über die Länge unterschiedlichen elastischen Eigenschaften, der nach der Implantation der Prothese seitlich zwischen die Wirbelkörper eingeschoben und an Prothesenkomponenten, an künstliche Wirbelendplatten oder den betroffenen Wirbelkörpern fixiert wird.

Die Bewegungsbegrenzenden Mittel können gemäß einer weiteren Ausgestaltung der Erfindung durch entsprechende Formgebungen der relativ zueinander beweglichen Prothesenkomponenten erreicht werden. Am Beispiel des Halbkugel-Pfannesystems lässt sich diese Lösung durch asymmetrische Formengebungen der Halbkugel und/oder Pfanne erreichen.

Die Bewegungsbegrenzende Funktion kann auch zumindest teilweise durch elastische Bänder, wie Zuggurtungsbänder übernommen werden, die den Bandscheibenbereich überbrücken und unterschiedliche Zugkräfte die notwendigen gezielten Bewegungsbegrenzungen hervorrufen. Die Bänder werden je nach Prothesenausführung entweder an Prothesenteilen, an künstlichen Wirbelendplatten oder an beiden angrenzenden oder weiteren Wirbelkörpern befestigt.

Das elastische Band kann vorzugsweise einen Dämpfer aufnehmen oder mit einem integrierten Dämpfer ausgebildet sein, wobei der Dämpfer unbefestigt zwischen z.B. Wirbelendplatten liegt. Damit wird eine Dämpfung auf Druck und auf Zug bewirkt, wobei das Bewegungsbegrenzungssystem wenn nötig, leicht entfernt und ausgetauscht werden kann, indem lediglich die Schraubverbindung des elastischen Bandes gelöst wird.

Weitere Ausführungsmöglichkeiten bestehen in der Verwendung von elastischen Federn mit unterschiedlichen Federcharakteristiken.

Die Anwendung der erfindungsgemäßen Maßnahmen beschränkt sich nicht auf Kugel-Pfannen Bandscheibenprothesen. Sie sind auf alle gelenkigen Bandscheibenprothesen anwendbar. So kann beispielweise eine Bandscheibenprothese direkt aus einem elastischen Material bestehen, das richtungsabhängig unterschiedliche elastische Eigenschaften hat, zwischen zwei Wirbelendplatten angeordnet ist und durch mindestens eine mit einer Wirbelendplatte verbundene Umrandung bzw. Schürze in der erforderlichen Lage löse gehalten wird. Das elastische Material kann aber auch mit einer Wirbelendplatte z.B. durch Verklebung verbunden sein, während die zweite Wirbelendplatte eine Umrandung oder Schürze aufweist.

Um die individuelle Wirkung für den jeweiligen Patienten zu erreichen, kann gemäß einer weiteren Ausführung der Erfindung eine Kombination von vorstehend beschriebenen Bewegungsbegrenzungsmittel eingesetzt werden.

Eine vorteilhafte Ausgestaltung der Erfindung besteht darin, dass die Bewegungsbegrenzungsmittel lösbar mit Prothesenkomponenten befestigt werden, wodurch in Revisionen die Mittel ohne die Prothese selber entfernen zu müssen leicht ausgetauscht werden können. Derartige eingriffe sind beispielweise notwendig, wenn für den Patienten eine neue Auslegung der einzelnen Bewegungsbegrenzungen erforderlich ist.

Eine weitere Ausgestaltung der Erfindung sieht künstliche Wirbelendplatten vor, die auf Dauer jeweils an eines der angrenzenden Wirbelkörper mittels Verklebung, Verschraubung, Krampen, etc. auf Dauer fixiert sind. Die gelenkige Prothesenkomponente und ggf. die Bewegungsbegrenzungsmittel werden lösbar mit den Wirbelendplatten, z.B. durch Verschraubung befestigt. Auf diese Weise reduziert sich eine Nachoperation, bei der die Prothesenkomponente oder Bewegungsbegrenzungsmittel ausgetauscht werden müssen, auf einen mechanischen Wechsel der Elemente. Hier wird also bei einem Revisionseingriff das Prothesenmodul und/oder die Bewegungsbegrenzungsmittel ausgetauscht, während die künstlichen Wirbelendplatten im implantierten Zustand verbleiben. Dadurch wird das Knochenmaterial geschont und der Eingriff bei einer Revision -wird deutlich einfacher und zeitlich verkürzt.

Einen weiteren Vorteil bieten genormte Wirbelendplatten, denen genormte Prothesenmodule unterschiedlicher Auslegung, Gestaltung und Typen zugeordnet werden. So ist ein einfacher Typenaustausch möglich, wenn z.B. bei einem Patienten ein Austausch der ursprünglich eingesetzten gelenkigen Prothesenkomponente durch eine einer anderen Ausgestaltung erforderlich ist.

Die Erfindung wird anhand von in der Zeichnung schematisch dargestellten Ausführungsbeispielen näher erläutert.

Es zeigen:
Fig.1 Eine Dorsalansicht von zwei Wirbelkörpern und einer Bandscheibenprothese mit Halbkugel-Pfannen-Technik im Schnitt,
Fig.2 Eine Seitenansicht eines Rückgrades mit mehreren Bandscheibenprothesen,
Fig.3 bis 6b je ein weiteres Ausführungsbeispiel der Erfindung.

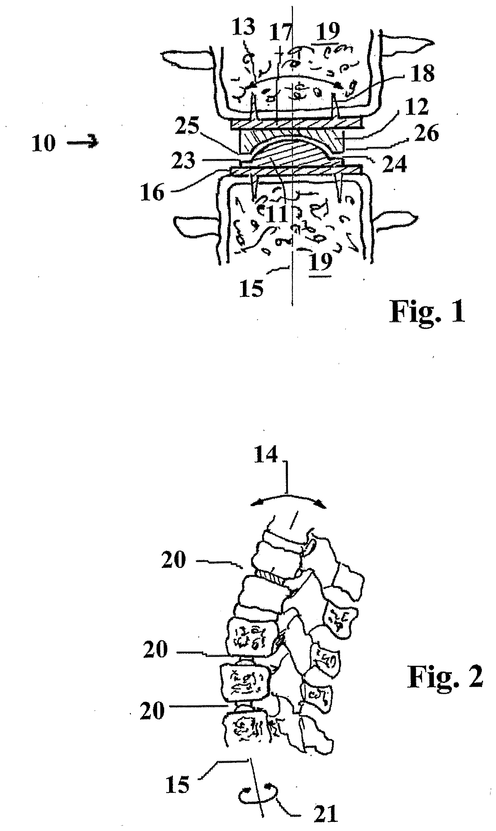

Fig.1 zeigt eine Bandscheibenprothese 10 bestehend aus einer Halbkugel 11 und einer mit ihr zusammenwirkenden Pfanne 12 im Längsschnitt. Die Halbkugel 11 und die Pfanne 12 werden jeweils von einer Wirbelendplatte 16 bzw. 17 getragen, die ihrerseits über Stifte oder Krampen 18 mit dem jeweiligen Wirbelkörper 19 verbunden werden. Eine Prothese dieser Art ist dreidimensional gelenkig, sie ist in Seitenneigung 13 und Rotation 21 um die Hochachse 15 beweglich. Außerdem erlaubt sie die in Fig.2 dargestellte Beuge-Extensionsbewegung 14.

Um die freie Beweglichkeit der Prothese an die Bedürfnisse des Patienten einzuschränken, werden Maßnahmen ergriffen, die die drei Bewegungsrichtungen 13, 14 und 21 je individuell begrenzen.

Gemäß Fig. 1 ist eine formgebende Maßnahme gezeigt, die die Seitenneigung z.B. nach rechts stärker begrenzt als nach links. Hierfür ist die Halbkugel 11 links mit einer niedrigen und rechts mit einer höheren Stufe 23 bzw. 24 ausgebildet, so dass bei einer Seitenneigung 13 durch den Patienten, die linke Pfannenkante 25 einen längen Weg ausführen kann als die rechte Pfannenkante 26. Um Beschädigungen und harte Stöße zu vermeiden, können die Stufen 23, 24 und/oder die Pfannenkanten 25,26 mit in der Zeichnung nicht dargestellten elastischen Auflagen beschichtet sein.

In ähnlicher Weise können Stufen dieser Art in der Ebene senkrecht zur Zeichnungsebene der Fig.1 für die Begrenzung der Beuge-Extensionsbewegung vorgesehen werden.

Es ist durchaus bei Patienten möglich, dass die Beweglichkeit eines implantierten Bandscheibenersatzes in Laufe von Jahren verändert werden muss. Es ist deshalb von Vorteil, wenn die Bandscheibenprothese mit Begrenzungsmitteln einen modularen Aufbau hat, bei dem künstliche Wirbelendplatten 16,17 auf Dauer mit den Wirbelkörpern 19 verbunden und die Halbkugel 11 sowie die Pfanne 12 lösbar (nicht in Fig. 1 dargestellt) an die künstlichen Wirbelendplatten 16,17 befestigt werden. Dazu können Bajonett- oder Schwalbenschwanzverbindungen zwischen Gelenkmodul 11,12 und künstlichen Wirbelendplatten 16,17 vorgesehen sein. Oder die Wirbelendplatten 16, 17 sind zu dem Zweck mit Schraubbohrungen ausgestattet.

In der Fig. 2 ist lediglich eine Seitenansicht eines Rückgrades mit mehreren Bandscheibenprothesen 20 dargestellt.

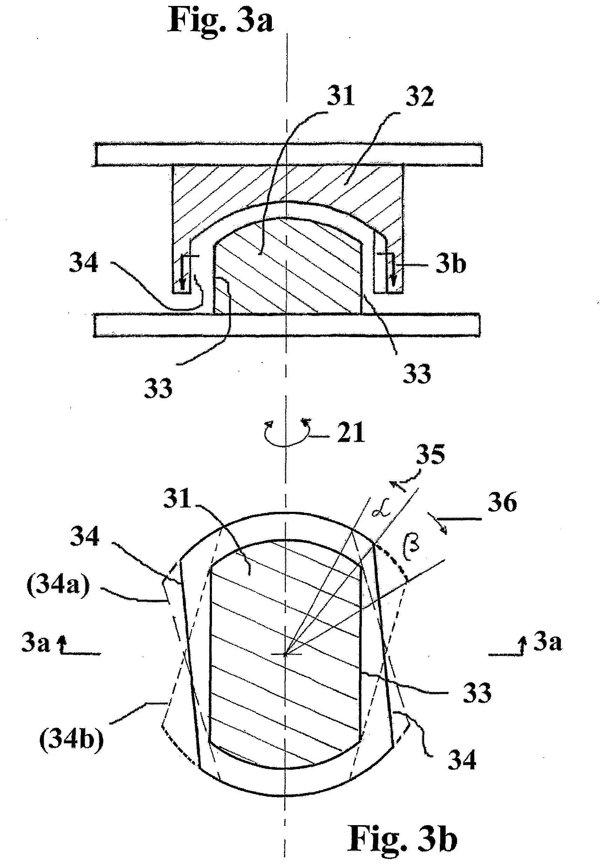

Eine formgebende asymmetrische Maßnahme für die Begrenzung der Rotationsbewegung 21 ist in Fig.3a im Teillängsschnitt und 3b im Querschnitt dargestellt. Die Halbkugel 31 der Bandscheibenprothese 30 hat zwei parallele Abflachungen 33 und die Pfanne 32 ebene, parallel zueinander verlaufende Verengungen 34, die jedoch in Ruhestellung der Prothese 30 schräg zu den Abflachungen 33 der Halbkugel verlaufen. Dieser Ruhezustand ist mit den durchgezogenen Linien der Verengungen 34 in Fig.3b dargestellt. Damit ist durch Formschluss die Rotationsbeweglichkeit 21 auf ein vorbestimmtes Maß reduziert. Die Schrägstellungen der ebenen Flächen 33 und 34 zueinander bewirken unterschiedliche maximale Rotationswinkel α,β nach links bzw. nach rechts. Bei einer Drehung des Patienten nach links (Pfeil 35) wird die Prothese einen kleineren Drehwinkel α als nach rechts (Pfeil 36, Winkel β) erlauben. Die Positionen der Pfanne 32 in den Drehbewegungsgrenzen 34a und 34b sind in Fig.3b gestrichelt dargestellt.

Bewegungsbegrenzungen durch Formschluß sind nicht auf die hier beschriebenen Ausführungen beschränkt. Jede andere Formgebung, Nut und Feder, Vorsprünge, etc., die geeignet sind, die Beweglichkeit einzuschränken, ist Gegenstand dieser Erfindung. Ebenfalls beschränkt sich die Erfindung nicht auf Bandscheibenprothesen in Kugel/Halbkugel-Pfannen Techniken. Die Erfindung lässt sich an diesem Beispiel besonders übersichtlich darstellen.

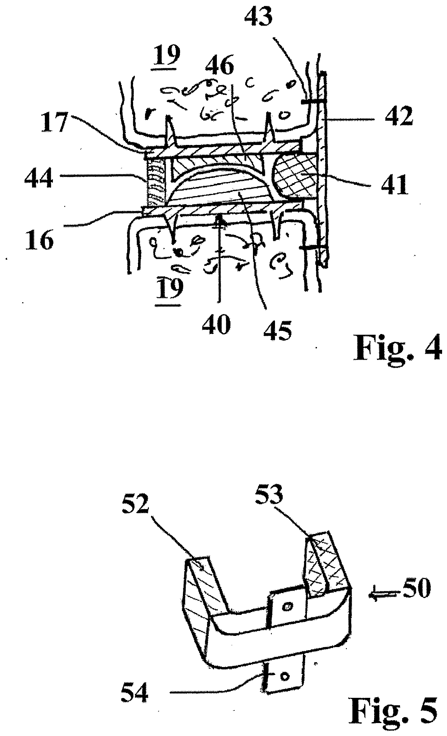

Eine Ausführung mit elastischen Bewegungsbegrenzungs-Mitteln sind in Fig.4 und 5 gezeigt. In Fig.4 ist eine Bandscheibenprothese 40 mit einem Puffer bzw. Dämpfer 41 gezeigt, der an einer Seite der Prothese 40 zwischen die künstlichen Wirbelendplatten 16,17 hineinragt und über ein elastisches Band 42, das an den Wirbelkörpern 19 mittels Schrauben 43 befestigt ist, gehalten wird. Auf der gegenüberliegenden Seite der Prothese 40 ist als weiteres Beispiel eine Zug- und/oder Druckfeder 44 dargestellt.

In der Regel werden zu beiden Seiten entweder Dämpfer 41 oder Federn 44 mit gleichen oder unterschiedlichen elastischen Eigenschaften eingesetzt. Es ist natürlich auch möglich, unterschiedliche Begrenzungsmittel, wie z.B. in Fig. 4 dargestellt, einzusetzen. Das elastische Band 42, wie z.B. ein Zuggurtungsband, wird ebenfalls zur Bewegungsbegrenzung einbezogen und lässt sich anstelle über die Schrauben 43 an den Wirbelkörpern 19 an den künstlichen Wirbelendplatten 16,17 fixieren.

Dämpfer 41 und Federn 44, dienen zu beiden Seiten der Gelenckomponente 45,46 gegenüberliegend angeordnet, zur Begrenzung der Beugungs-Extensionsbewegung oder der Seitenneigungen. Um beide Bewegungsrichtungen zu begrenzen werden in der Ebene quer zur Zeichnungsebene ebenfalls derartige oder ähnliche Mittel eingesetzt, wobei alle oder nur zwei gegenüberliegende. Mittel unterschiedliche Eigenschaften zur ungleichen Bewegungsbegrenzung haben. Die mit den Wirbelendplatten 16,17 verbundenen Federn 44 und das elastische Band 42 üben auch Zugkräfte aus, die in die Auslegung der Beweglichkeit einbezogen werden.

Über die Elastizität des Dämpfers 41, wenn diese mit den Verbindungsplatten 16,17 z.B. durch Verklebung verbunden sind, und der Zugkraft des Bandes 42 wird zusätzlich die Rotationsbeweglichkeit in Grenzen gehalten. Federn 44 können durch entsprechende Auslegung über die Scherkräfte auch gezielten Einfluss auf die Rotation um die Hochachse ausüben.

Die Platten 16,17 können auch als von der Gelenkprothese 45,46 getrennte künstliche Wirbelendplatten 16, 17 angesehen werden, die so an den Wirbelkörpern angebracht sind, dass sie als Halterung für diverse Komponenten dienend auf Dauer implantiert bleiben. Das Zuggurtungsband kann in diesem Fall anstelle am Wirbelknochen an die künstliche Wirbelendplatten 16, 17 angeschraubt werden. Natürlich sind in so einem Fall, die Gelenkteile 45,46 der Prothese 40 ebenfalls lösbar mit den künstlichen Wirbelendplatten 16, 17 verbunden.

Zweckmäßig ist, wenn die für die Bewegungsbegrenzung getrennte Mittel 41,42,44,50, wie in Fig. 4 und 5 gezeigt, verwendet werden, die zudem lösbar mit künstlichen Wirbelendplatten 16, 17 verbunden werden. Bei einer notwenigen Änderung der Beweglichkeit ist dann in einem erneuten chirurgischen Eingriff lediglich ein Austausch der Mittel 41,42,44,50 nötig, indem die auszuwechselnden Mitteln von den künstlichen Wirbelendplatten 16, 17 gelöst und die neuen eingesetzt und daran befestigt werden, ohne die gesamte Bandscheibenprothese 40 auswechseln zu müssen.

Anstelle mehrerer Dämpfer stellt ein einziger Dämpfer 50, der die Gelenkstelle 45,46 der Bandscheibenprothese 40 zumindest teilweise umgibt, ein einfaches Mittel zur Bewegungsbegrenzung dar. Ein Beispiel hierzu ist in Fig.5 dargestellt, das in einem U-förmigen Dämpfer 50 besteht, der von der Eingriffsseite zwischen die künstlichen Wirbelendplatten 16,17 eingeschoben und mittels Befestigungsnasen 54 an die Seitenkanten der Verbindungsplatten 16,17 angeschraubt wird. Die Geometrie des annähernd U-förmigen Dämpfers 50 wird an die Bandscheibenprothese angepasst, z.B. sind halbkreisförmige elastische Dämpfer möglich. Um unterschiedlichen Einfluß auf die Beweglichkeit in verschiedenen Richtungen zu haben, wird der Dämpfer 50 über die Länge mit unterschiedlichen Charakteristiken ausgestattet. So können beispielweise die Schenkel 52 und 53 des U-förmigen Dämpfers 50 mit elastischen Eigenschaften ausgestattet werden, die sich von denen des Verbindungsstegs 51 oder auch untereinander unterscheiden.

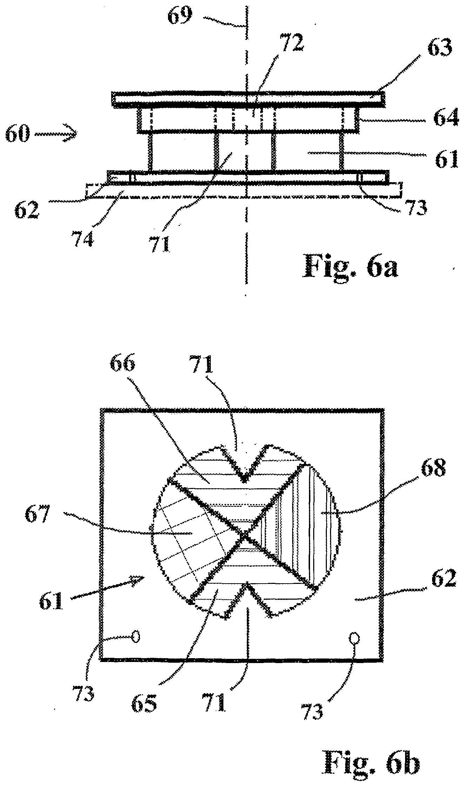

Figuren.6a und 6b zeigen ein Beispiel in Seitenansicht bzw. im Querschnitt, bei dem die Bandscheibenprothese 60 im wesentlichen aus einem elastischen Zylinder 61 besteht, der mit der unteren Platte 62 z.B. durch kleben verbunden ist. Die obere Platte 63 weist eine hülsenförmige Schürze 64 auf, die den elastischen Zylinder 61 zentrierend umgreift.

Der elastische Zylinder 61 besteht aus vier Sektoren 65-68 unterschiedlicher elastischer Eigenschaften, wobei beispielsweise für die Seitenneigungen zwei gegenüberliegende eigenschaftsgleiche Sektoren 65,66 vorgesehen sind. Zur unterschiedlichen Begrenzung der Beuge- und Extensionsbewegung sind die beiden übrigen Sektoren 67,68 mit unterschiedlichen elastischen Charakteristiken ausgelegt. Es sind auch viele unterschiedliche Materialpaarungen mit Kunststoffen, Metall, Keramik bzw. diese untereinander möglich.

Zur Begrenzung der Rotation um die Hochachse 69 sind in gegenüberliegenden Sektoren 65,66 je eine keilförmige Nut 71 parallel zur Hochachse 69 eingearbeitet, in die sich je eine keilförmige Nase 72 an der Schürzeninnenseite mit entsprechendem Spiel hineinragt. Anstelle der Keilform kann jede andere Form gewählt werden, z.B. Nut und Nase mit quadratischem Querschnitt.

Das Beispiel gemäß Fig. 6 kann dahingehend variiert werden, dass beispielsweise die obere Platte 63 mit der Schürze 64 eine Baueinheit bildend als künstliche Wirbelendplatte 63,64 am Wirbelkörper verankert wird und als Halterung dient. Am gegenüberliegenden Wirbelkörper wird eine gestrichelt dargestellte, ebene Wirbelendplatte 74 ebenfalls auf Dauer verankert. Die Prothesenkomponente 61,62 wird zwischen die beiden Wirbelendplatten 63,74 zwischengelegt und mit der unteren künstlichen Wirbelendplatte 74 lösbar verbunden. Dazu sind beispielsweise in der plattenförmigen Prothesenkomponente 74 Schrägbohrungen 73 und in der künstlichen Wirbelendplatte 74 Schraubbohrungen (in der Zeichnung nicht dargestellt) für eine Schraubverbindung vorgesehen.

Die als Halterungen dienenden künstlichen Wirbelendplatten 63,64 und 74 können weitere Elemente z.B. zur Bewegungsbegrenzung, aufnehmen, die ebenfalls lösbar angebracht werden. Im Beispiel nach Fig. 6 kann die Rotation um die Hochachse 69 auch durch elastische Bänder beispielsweise begrenzt werden, die entsprechend an die beiden künstlichen Wirbelendplatten 63,64 und 74 angeschraubt werden. In diesem Fall entfallen die Nuten 71 und Nasen 72.

## Patentansprüche

1. Bandscheibenprothese bestehend aus gelenkigen Prothesenkomponenten und Mitteln, die die Beweglichkeit begrenzen, wobei die Begrenzungsmittel derart ausgebildet sind, dass sie die Beuge-Extensionsbewegung und/oder die Seitenneigung (13) und/ oder die Rotation um die Hochachse asymmetrisch begrenzen, wobei ein Halbkugel-Pfannesystem als gelenkige Prothesenkomponente vorgesehen ist, wobei die bewegungsbegrenzenden Mittel durch asymmetrische Formgebungen (23,24) der Halbkugel (11) und/oder Pfanne (12) ausgebildet sind, derart dass die Formgebungen für jede Bewegungsrichtung (14,15, 21) gezielt und individuell an den Patienten anpassbar sind, **dadurch gekennzeichnet, dass** die Halbkugel (11) mit einer niedrigen Stufe (23) und mit einer höheren Stufe (24) ausgebildet ist, die mit der Pfannenkante die Seitenneigung der Bandscheibenprothese begrenzt.

2. Bandscheibenprothese bestehend aus gelenkigen Prothesenkomponenten und Mitteln, die die Beweglichkeit begrenzen, wobei die Begrenzungsmittel derart ausgebildet sind, dass sie die Beuge-Extensionsbewegung und/oder die Seitenneigung (13) und/ oder die Rotation um die Hochachse asymmetrisch begrenzen, wobei ein Halbkugel-Pfannesystem als gelenkige Prothesenkomponente vorgesehen ist, wobei die bewegungsbegrenzenden Mittel durch asymmetrische Formgebungen (23,24) der Halbkugel (11) und/oder Pfanne (12) ausgebildet sind, derart dass die Formgebungen für jede Bewegungsrichtung (14,15, 21) gezielt und individuell an den Patienten anpassbar sind, **dadurch gekennzeichnet, dass** die Halbkugel (11) mit einer niedrigen Stufe (23) und mit einer höheren Stufe (24) ausgebildet ist, die mit der Pfannenkante die Beuge-Extensionsbewegung der Bandscheibenprothese begrenzt.

3. Bandscheibenprothese nach Anspruch 1 oder 2, **dadurch gekennzeichnet, dass** die Stufen 23, 24 und/oder die Pfannenkanten 25,26 mit elastischen Auflagen beschichtet sind.

4. Bandscheibenprothese nach Anspruch 1, 2 oder 3, **dadurch gekennzeichnet, dass** mindestens ein elastisches Band (42), das an mindestens zwei Wirbelkörpern befestigbar ist, vorgesehen ist, die Zugkräfte ausüben.

5. Bandscheibenprothese nach Anspruch 4, **dadurch gekennzeichnet, dass** das bzw. die elastischen Bänder (42) zur Aufnahme und Teilfixierung von Bandscheibenprothesen oder Teile (41) davon ausgebildet sind.

6. Bandscheibenprothese nach einem der vorhergehenden Ansprüche, **dadurch gekennzeichnet, dass** teilweise Zuggurtungsbänder (42) als Bewegungsbegrenzungsmittel vorgesehen sind.

## Claims

1. Intervertebral disc prosthesis consisting of articulated prosthesis components and means limiting the movability, wherein the limiting means are configured in such a way that they asymmetrically limit the flexion-extension movement and/or the lateral inclination (13) and/or the rotation about the vertical axis, wherein a hemisphere-cup system is provided as an articulated prosthesis component, wherein the movement-limiting means are provided by asymmetric configurations (23, 24) of the hemisphere (11) and/or cup (12) in such a way that the configurations for each direction of movement (14, 15, 21) can be specifically and individually adapted to the patient, **characterized in that** the hemisphere (11) is formed with a low step (23) and with a higher step (24) which, together with the cup edge, limits the lateral inclination of the intervertebral disc prosthesis.

2. Intervertebral disc prosthesis consisting of articulated prosthesis components and means limiting the movability, wherein the limiting means are configured in such a way that they asymmetrically limit the flexion-extension movement and/or the lateral inclination (13) and/or the rotation about the vertical axis, wherein a hemispherecup system is provided as an articulated prosthesis component, wherein the movement-limiting means are provided by asymmetric configurations (23, 24) of the hemisphere (11) and/or cup (12) in such a way that the configurations for each direction of movement (14, 15, 21) can be specifically and individually adapted to the patient, **characterized in that** the hemisphere (11) is formed with a low step (23) and with a higher step (24) which, together with the cup edge, limits the flexion-extension movement of the intervertebral disc prosthesis.

3. Intervertebral disc prosthesis according to claim 1 or 2, **characterized in that** the steps 23, 24 and/or the cup edges 25, 26 are coated with elastic linings.

4. Intervertebral disc prosthesis according to claims 1, 2 or 3, **characterized in that** at least one elastic band (42) which is attachable to at least two vertebral bodies and exerts tensile forces is provided.

5. Intervertebral disc prosthesis according to claim 4, **characterized in that** the elastic band(s) (42) are provided for receiving and partially fixing intervertebral disc prostheses or parts (41) thereof.

6. Intervertebral disc prosthesis according to one of the preceding claims, **characterized in that** in part tension belts (42) are provided as movement-limiting means.

## Revendications

1. Prothèse de disque intervertébral constituée de composants de prothèse articulés et de moyens, lesquels limitent la mobilité, dans laquelle les moyens limiteurs sont conçus de telle sorte qu'ils limitent de manière asymétrique le mouvement de flexion-extension et/ou l'inclinaison latérale (13) et/ou la rotation autour de l'axe vertical, dans laquelle un système de cupule et d'hémisphère est fourni en tant que composant de prothèse articulé, dans laquelle les moyens limitant le mouvement sont conçus par des configurations (23, 24) asymétriques de l'hémisphère (11) et/ou de la cupule (12) de telle sorte que les configurations visent chaque direction de mouvement (14, 15, 21) et peuvent être adaptées au patient de manière individuelle, **caractérisée en ce que** l'hémisphère (11) est conçu de manière à comporter un étage inférieur (23) et un étage supérieur (24), lesquels limitent, avec le bord de cupule, l'inclinaison latérale de la prothèse de disque intervertébral.

2. Prothèse de disque intervertébral constituée de composants de prothèse articulés et de moyens, lesquels limitent la mobilité, dans laquelle les moyens limiteurs sont conçus de telle sorte qu'ils limitent de manière asymétrique le mouvement de flexion-extension et/ou l'inclinaison latérale (13) et/ou la rotation autour de l'axe vertical, dans laquelle un système de cupule et d'hémisphère est fourni en tant que composant de prothèse articulé, dans laquelle les moyens limitant le mouvement sont conçus par des configurations (23, 24) asymétriques de l'hémisphère (11) et/ou de la cupule (12) de telle sorte que les configurations visent chaque direction de mouvement (14, 15, 21) et peuvent être adaptées au patient de manière individuelle, **caractérisée en ce que** l'hémisphère (11) est conçu de manière à comporter un étage inférieur (23) et un étage supérieur (24), lesquels limitent, avec le bord de cupule, le mouvement de flexion-extension de la prothèse de disque intervertébral.

3. Prothèse de disque intervertébral selon la revendication 1 ou 2, **caractérisée en ce que** les étages 23, 24 et/ou les bords de cupule 25, 26 sont revêtus de couches élastiques.

4. Prothèse de disque intervertébral selon la revendication 1, 2 ou 3, **caractérisée en ce qu'**au moins une bande (42) élastique, laquelle peut être fixée à au moins deux corps vertébraux, est fournie, laquelle exerce des forces de traction.

5. Prothèse de disque intervertébral selon la revendication 4, **caractérisée en ce que** l'au moins une bande élastique (42) est conçue pour recevoir et pour sécuriser partiellement des prothèses de disque intervertébral ou des parties (41) de celles-ci.

6. Prothèse de disque intervertébral selon l'une quelconque des revendications précédentes, **caractérisée en ce que** des membrures de tension (42) sont partiellement fournies en tant que moyens de limitation de mouvement.
